# EUROPÄISCHE PATENTANMELDUNG

(11) **EP 0 543 259 A1**
(43) Veröffentlichungstag der Anmeldung: **26.05.1993**
(21) Anmeldenummer: 92119236.5
(22) Anmeldetag: 11.11.1992
(51) Int. Cl.: A61M 5/168

(54) **Mehrfachinfusionssystem**

(30) Priorität: 18.11.1991 DE 4137837
(71) Anmelder: Fresenius AG, D-61350 Bad Homburg (DE)
(72) Erfinder: Neuder, Klaus, Dr., W-6053 Obertshausen (DE)
(74) Vertreter: Fuchs, Luderschmidt & Partner Patentanwälte

(57) **Zusammenfassung**

Um nicht nur einen Fluidstopp in der Patientenleitung (41) zu erkennen sondern auch Störungen im Bereich der einzelnen Infusionseinrichtungen (10, 20, 30), die aus Schwerkraftinfusionseinrichtungen und/oder Druckinfusionsapparaten bestehen können, ist eine Überwachungseinrichtung (1) mit mindestens einem Alarmeingang (2, 3, 4) vorgesehen, wobei ein Alarmeingang (3) mit einem der Schwerkraftinfusionseinrichtung (10) zugeordneten Tropfendetektor (12) und/oder ein Alarmeingang (2) mit einem Alarmausgang (22, 32) des Druckinfusionsapparates (20, 30) verbunden ist. Bei Detektion einer Abweichung von einer vorgegebenen Tropfrate oder beim Anliegen eines Alarmsignales eines Druckinfusionsapparates (20, 30) desaktiviert die Überwachungseinrichtung (1) alle noch nicht desaktivierten Infusionseinrichtungen (10, 20, 30) durch Schließen des Ventils (14) und/oder Abschalten des Druckinfusionsapparates (20, 30). Vorzugsweise erfolgt das Desaktivieren der Infusionseinrichtung (10, 20, 30), wenn eine innerhalb einer vorgegebenen Zeitspanne anhaltende Abweichung von einem vorgegebenen Tropfratenbereich vorliegt. Über einen zusätzlichen Alarmeingang (4) kann eine externe Einrichtung zum Ausschalten eines fremden Alarmsignales angeschlossen sein.

## Beschreibung

Die Erfindung betrifft eine Vorrichtung zur dosierten, kontinuierlichen, gleichzeitigen Infusion von mehreren Infusions- oder Medikamentenlösungen gemäß dem Oberbegriff von Anspruch 1.

Die moderne Intensivtherapie benutzt in zunehmendem Maße Mehrfachinfusionsvorrichtungen zur künstlichen, parenteralen Ernährung, zur kontinuierlichen bzw. genau dosierten Applikation von Medikamenten und in neuerer Zeit auch zur geregelten Zufuhr hochwirksamer Medikamente in Abhängigkeit von physiologischen Parametern. In der Regel werden drei bis vier unterschiedliche Medikamenten- bzw. Infusionslösungen mit Hilfe mehrerer Druckinfusionsapparate und/oder Schwerkraftinfusionseinrichtungen über einen Zugang zum Blutkreislauf des Patienten appliziert, der gewöhnlich aus einem zentralvenösen Katheter besteht.

Bei der Kombination von Druckinfusionsapparaten und Schwerkraftinfusionseinrichtungen kommt es bei einem Fluidstopp in der Patientenleitung zu einer Förderung von Infusion aus dem Druckinfusionsapparat in die Schwerkraftinfusionseinrichtung, da das Druckerkennungssystem des Druckinfusionsapparates erst bei einem bestimmten Staudruck anspricht. Der Infusionsstopp bleibt daher zunächst unerkannt.

Wird die Förderleitung des Druckinfusionsapparates unterbrochen (z.B. durch einen versehentlich geschlossenen Dreiwegehahn), so pumpt der Druckinfusionsapparat solange weiter, bis das Druckerkennungssystem des Druckinfusionsapparates anspricht und sich der Druckinfusionsapparat abschaltet. Aufgrund der Verformbarkeit der Infusionsleitungen kommt es dann aber zur Ausbildung eines Speichervolumens, das sich beim Öffnen des Dreiwegehahnes bzw. nach der Beseitigung der Unterbrechung schlagartig in den Patienten entleeren kann.

Das irrtümliche Verschließen eines Dreiwegehahnes kommt relativ häufig vor, da z.B. zur Probenentnahme, aber auch zur Messung des zentralvenösen Druckes die Verbindungshähne zu den Pumpen kurzzeitig geschlossen werden müssen.

Es gibt zwar gewisse Verhaltensmaßregeln und Vorschriften, nach denen die vorgenannten Gefährdungen vermieden werden sollen, jedoch verlangen diese ein großes Konzentrationsvermögen des Anwenders bzw. sie sind mit erhöhten Kosten verbunden. So gilt es beispielsweise nicht als fachgerecht, eine Pumpeninfusion mit einer Schwerkraftinfusion zu kombinieren. Dies führt wiederum dazu, daß selbst dort eine Infusionspumpe eingesetzt werden muß, wo dies von der Genauigkeitsanforderung an die Förderung des Medikamentes bzw. der Infusionslösung nicht gerechtfertigt ist, was zu erhöhten Kosten führt.

Um einen Teil der genannten Nachteile zu vermeiden, sind sogenannte Mehrfachinfusionssysteme entwickelt worden. Aus der DE-PS 33 29 977 ist eine Vorrichtung zur dosierten Infusion von Lösungen aus mehreren Infusionsbehältern bekannt. Mehrere Schwerkraftinfusionseinrichtungen mit Tropfenzähler sind über Ablaufleitungen, in die Klemmventile eingebaut sind, an einer Sammelleitung angeschlossen, die zum Patienten führt. In dieser Patientenleitung ist stromab zunächst ein Durchflußsensor und danach eine Förderpumpe angeschlossen. Alle Ventile und der Durchflußsensor sowie die Pumpe sind mit einer Steuereinheit verbunden.

Die vom Arzt zu wählende Infusionsrate für die einzelnen Lösungen werden in dem Steuergerät in proportionale Tropfenzahlen umgerechnet. Jedes Ventil bleibt so lange geöffnet, bis die diesen Kanal zugeordnete Tropfenzahl erreicht ist. Danach wird das Ventil geschlossen und das Ventil des nächsten Kanals geöffnet. Es werden jeweils bestimmte Tropfenzahlen ermittelt und der Sammelleitung zugeführt, bis eine Umschaltung erfolgt. Diese Vorrichtung hat eine Reihe von Nachteilen. Die Infusion erfolgt sequentiell, d.h. die Förderung der einzelnen Lösungen erfolgt nicht kontinuierlich. Außerdem ist die Förderung eines hochwirksamen Medikamentes mit geringerer Rate und gleichzeitig beispielsweise einer parenteralen Nährlösung mit hoher Rate nicht möglich. Ein weiterer Nachteil besteht darin, daß die einzige Pumpe mit konstanter Förderrate arbeitet und ein Defekt an der Pumpe zu einer unkontrollierten Infusion führt.

Aus der DE 28 55 713 C2 ist zwar eine Vorrichtung zur Infusion von Lösungen aus mehreren Infusionsflaschen bekannt, bei der eine sogenannte Wächtereinrichtung stromab einer Pumpe angeordnet ist, jedoch ist diese Wächtereinrichtung zum einen in ihrer Funktion bis auf die Angabe der Erfassung von Luftblasen nicht näher erläutert und zum anderen dient sie lediglich zur Signalisierung irgendwelcher Störungen. Sie ist jedoch nicht dazu geeignet, einen Steuervorgang auszulösen, da sie nicht mit der Steuereinheit der aus dieser Druckschrift bekannten Vorrichtung verbunden ist.

Aus der EP 0 343 501 A2 ist ein Mehrfachinfusionssystem mit mehreren Druckinfusionsapparaten und Schwerkraftinfusionseinrichtungen bekannt, wobei ein Durchflußelement stromab des Druckinfusionsapparates angeordnet ist und eine Steuereinheit vorgesehen ist, die bei Erkennen eines Fluidstopps durch das Durchflußelement durch Schließen der Ventile der Schwerkraftinfusionseinrichtungen und Abschalten der Pumpen der Druckinfusionsapparate das Mehrfachinfusionssystem desaktiviert. Diese Vorrichtung bietet zwar den Vorteil, daß eine Unterbrechung des Durchflusses erkannt wird, noch ehe ein Druckaufbau von der oder den Druckinfusionsapparaten festgestellt wird, jedoch werden Störungen im Bereich der einzelnen Infusionseinrichtungen von der Steuereinrichtung nicht erkannt und berücksichtigt. Insbesondere werden Leckagen oder Verschlüsse in den einzelnen Förderleitungen der jeweiligen Infusionseinrichtungen bzw. Störungen der Druckinfusionsapparate nicht erkannt, weil dadurch unter Umständen der Durchfluß durch die Patientenleitung nicht wesentlich beeinflußt wird. So kann beispielsweise die Mehrfachinfusionsvorrichtung eine Spritzenpumpe enthalten, die lediglich eine kleine Menge zur Gesamtinfusionsmenge beisteuert. Ein Abknicken des zugeordneten Förderschlauches würde die Zufuhr der entsprechenden Medikamentenlösung unterbrechen, ohne daß dies erkannt wird, weil sich die Durchflußmenge durch das in der gemeinsamen Patientenleitung angeordnete Durchflußelement aufgrund des geringen Anteils an der Gesamtinfusionsmenge nicht merklich ändert. Da insbesondere bei hochwirksamen Medikamenten die Mischung der Einzelkomponenten exakt aufeinander abgestimmt sein muß, kann beim Ausfall eines Medikamentes dies zu gefährlichen Folgen für den Patienten führen. Auch kann in diesen Fällen eine Verschiebung der Medikamentendosierung eintreten, da der Ausfall einer Infusionslösung oder Medikamentenlösung durch ein stärkeres Abfließen beispielsweise aus einer Schwerkraftinfusionseinrichtung in gewissen Maßen kompensiert wird.

Es ist daher Aufgabe der Erfindung eine Mehrfachinfusionsvorrichtung zu schaffen, mit der nicht nur ein Fluidstopp in der Patientenleitung erkannt wird, sondern auch eine Störung im Bereich der einzelnen Infusionseinrichtungen detektiert wird und in diesen Fällen eine Fortsetzung der Infusion unterbunden wird.

Diese Aufgabe wird mit einer Vorrichtung gemäß den Merkmalen von Anspruch 1 gelöst.

Die erfindungsgemäße Vorrichtung kann ausschließlich Schwerkraftinfusionseinrichtungen, ausschließlich Druckinfusionsapparate oder eine Kombination aus Druckinfusionsapparaten und Schwerkraftinfusionseinrichtungen aufweisen. Die Überwachungseinrichtung weist mindestens einen Alarmeingang auf, der nicht wie im Stand der Technik an einem gemeinsamen in der Patientenleitung angeordneten Detektor angeschlossen ist, sondern mit den einzelnen Infusionseinrichtungen verbunden ist.

Wenn es sich um eine Mehrfachinfusionseinrichtung mit Schwerkraftinfusionseinrichtungen handelt, sind die Alarmeingänge jeweils mit denen den Schwerkraftinfusionseinrichtungen zugeordneten Tropfendetektoren verbunden. Ein weiterer Alarmeingang ist mit den Alarmausgängen der Druckinfusionsapparate verbunden. Gegebenenfalls kann noch ein weiterer Alarmeingang vorgesehen sein, an den eine externe Einrichtung zum Aufschalten einer fremden Alarmbedingung angeschlossen ist. Hierbei kann es sich beispielsweise um die Überwachung der Atmung des Patienten handeln, so daß beispielsweise bei einer Atemdepression ein entsprechendes Signal in der Überwachungseinrichtung eingeht, die sofort zu einem Stopp aller Infusionseinrichtungen führt.

An diesem zusätzlichen Alarmeingang kann auch ein externer Druckdetektor angeschlossen sein, der beispielsweise in der Patientenleitung angeordnet ist, wobei der Maximaldruck durch den Anwender vorgegeben werden kann.

Die Vorrichtung, die ausschließlich Schwerkraftinfusionseinrichtungen in Kombination mit Druckinfusionsapparaten vorsieht, überwacht das Tropfsignal von mindestens einer Schwerkraftinfusionseinrichtung überwacht wird. Falls es zu einem Fluidstopp in der Patientenzuleitung kommen sollte, ändert sich sofort die Tropfrate in der Schwerkraftinfusionseinrichtung. Gerade bei einer Kombination von einer Schwerkraftinfusionseinrichtung mit Druckinfusionsapparaten ändert sich zuerst die Tropfrate, bevor sich ein Druck aufbaut, der von einem Druckinfusionsapparat detektiert wird. Ein Fluidstopp wird daher zumindest genau so schnell erkannt, wie bei der bekannten Vorrichtung mit einem Durchflußelement in der Patientenleitung. Der Vorteil der erfindungsgemäßen Vorrichtung besteht darin, daß auf einen zusätzlichen Durchflußdetektor in der Patientenleitung verzichtet werden kann.

Da jedoch eine Verlagerung des Patienten einen unmittelbaren Einfluß auf die Tropfrate hat, ist gemäß einer besonderen Ausführungsform vorgesehen, daß die Überwachungseinrichtung erst dann die Ventile der Schwerkraftinfusionseinrichtungen schließt und die Druckinfusionsapparate durch Abschalten desaktiviert, wenn ein bestimmter Tropfratenbereich über eine bestimmte vorgegebene Zeit hinweg verlassen wird. Vorzugsweise kann eine Zeit zwischen 10 und 99 sec. gewählt werden. Der Tropfratenbereich kann vom Anwender in einem Bereich von ± 25%, ± 50%, ± 75% bezüglich eines Sollwertes eingestellt werden, wobei der Sollwert von der Überwachungseinrichtung automatisch nach einer bestimmten Zeit übernommen wird oder der Anwender den aktuellen Tropfratensollwert durch Tastendruck bestätigt. Beispielsweise ein Umdrehen des Patienten wird somit nicht fälschlicherweise als ein Fluidstopp oder ein sonstiger Defekt erkannt. Auch eine Leckage in der der Schwerkraftinfusionseinrichtung zugeordneten Förderleitung, beispielsweise im Bereich des Anschlusses an einer Dreiwegehahnbank wird sofort erkannt, weil dadurch die Tropfrate sich schlagartig erhöht.

Dadurch, daß die Druckinfusionsapparate mit ihrem Alarmausgang, der vorzugsweise der Personalrufanschluß ist, mit einem Alarmeingang der Überwachungseinrichtung verbunden sind, werden auch Störungen eines Druckinfusionsapparates sofort erkannt. In diesem Fall werden dann die Schwerkraftinfusionseinrichtungen mittels ihrer Ventile abgesperrt und die noch nicht desaktivierten Druckinfusionsapparate abgeschaltet.

Wenn die Mehrfachinfusionseinrichtung ausschließlich Druckinfusionsapparate aufweist, so wird ein Fluidstopp durch das Ansteigen des Druckes in den Druckinfusionsapparaten erkannt. Da alle Druckinfusionsapparate an die Überwachungseinrichtung angeschlossen sind, braucht nur ein Druckinfusionsapparat ein Druckerkennungssystem aufzuweisen, das beim Auftreten eines erhöhten Druckes sofort ein Signal an die Überwachungseinrichtung liefert, wodurch die noch nicht desaktivierten Druckinfusionsapparate sofort abgeschaltet werden.

Wenn alle Druckinfusionsapparate ein Druckerkennungssystem aufweisen, so ist der niedrigste Verschlußalarmdruck der angeschlossenen Druckinfusionsapparate die bestimmende Größe für den maximal entstehenden Druck bei der Mehrfachinfusion. Der Anwender braucht somit lediglich einen Druckinfusionsapparat hinsichtlich des Verschlußalarmdruckes sensitiv einzustellen.

Die Vorrichtung bietet somit weitaus wirksamere und bessere Überwachung der Infusion, wodurch die Sicherheit für den Patienten erhöht wird.

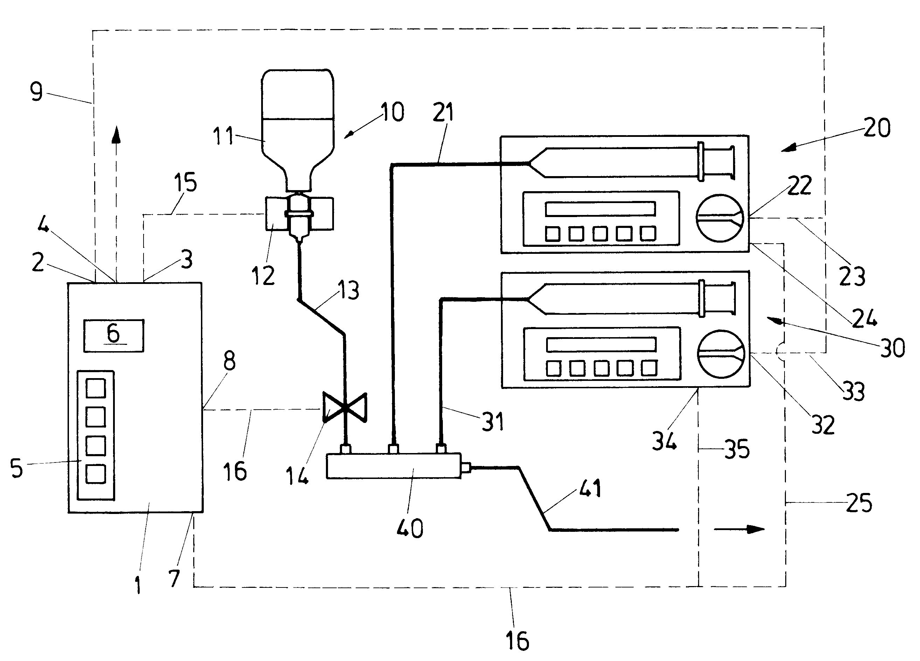

Eine beispielhafte Ausführungsform der Erfindung wird nachfolgend anhand der Figur erläutert.

In der Figur ist schematisch eine Mehrfachinfusionsvorrichtung dargestellt, die eine Schwerkraftinfusionseinrichtung 10 und einen ersten Druckinfusionsapparat 20 sowie einen zweiten Druckinfusionsapparat 30 jeweils in Gestalt von Spritzeninfusionspumpen umfaßt.

Die Schwerkraftinfusionseinrichtung 10 besitzt einen Vorratsbehälter 11 für die Infusions- oder Medikamentenlösung, unterhalb dem ein Tropfendetektor 12 in der zugeordneten Förderleitung 13 angeordnet ist. Die Förderleitung 13 mündet in eine Dreiwegehahnbank 40 und besitzt vor dem Anschluß an die Dreiwegehahnbank 40 ein steuerbares Ventil 14. Zwischen Tropfendetektor 12 und Ventil 14 ist eine Rollenklemme (nicht dargestellt) vorgesehen, mit der der Anwender die gewünschte Tropfrate einstellen kann.

Die Druckinfusionsapparate 20 und 30 sind mit ihren zugeordneten Förderleitungen 21 und 31 ebenfalls an die Dreiwegehahnbank 40 angeschlossen, von der die Patientenleitung 41 zum Patienten führt.

Weiterhin ist eine Überwachungseinrichtung 1 vorgesehen, die insgesamt drei Alarmeingänge 2, 3 und 4 aufweist. Der Alarmeingang 3 ist über eine Signalleitung 15 mit dem Tropfendetektor 12 verbunden. Der Signaleingang 2 ist über eine Signalleitung 9 mit den Signalleitungen 23 und 33 der Druckinfusionsapparate 20 und 30 verbunden, die an den Personalrufanschlüssen 22 und 32 angeschlossen sind.

Der dritte Alarmeingang 4 dient zum Anschluß eines externen Gerätes, das hier nicht dargestellt ist und zum Beispiel einen zusätzlichen Druckdetektor oder eine Atmungsüberwachungseinrichtung einschließen kann.

Die Überwachungseinrichtung 1 besitzt in dem hier gezeigten Beispiel zwei Steuerausgänge 7 und 8, wobei der Steuerausgang 7 über die Steuerleitung 16 mit den Steuerleitungen 25 und 35 verbunden ist, die an den Steuereingängen 24 und 34 der jeweiligen Druckinfusionsapparate 20 und 30 enden. Zur Eingabe einer Tropfrate bzw. eines Tropfratenbereiches und einer vorgebbaren Zeitspanne ist ein Eingabefeld 5 vorgesehen. Im Anzeigefeld 6 können die eingegebenen Werte abgelesen werden.

Wenn ein Fluidstopp beispielsweise in der Patientenleitung 41 auftritt, ändert sich als erstes die Tropfrate, die vom Tropfendetektor 12 ermittelt und von der Überwachungseinrichtung 1 erkannt wird. Wenn der vorgegebene Tropfratenbereich für eine vorgegebene Zeitspanne, die zwischen 10 und 99 sec eingestellt werden kann, überschritten wird, so wird das Ventil 14 durch ein entsprechendes Signal der Überwachungseinrichtung 1 geschlossen und die Druckinfusionsapparate 20 und 30 durch ein entsprechendes Signal über die Steuerleitungen 16, 25 und 35 abgeschaltet.

Sollte ein Druckinfusionsapparat 20 oder 30 nicht in der vorgesehenen Weise arbeiten, so wird dies von dem Druckinfusionsapparat erkannt und ein Signal über den jeweiligen Personalrufanschluß 22 bzw. 32 der Überwachungseinrichtung 1 mitgeteilt. Bei einem Alarmzustand schaltet sich der jeweilige Druckinfusionsapparat ab, aber der alarmfreie Druckinfusionsapparat bzw. auch die Schwerkraftinfusionseinrichtung laufen noch weiter. Um dies zu verhindern, schaltet die Überwachungseinrichtung 1 in einem solchen Fall sofort das Ventil 14 und stoppt den alarmfreien Druckinfusionsapparat.

Wenn beispielsweise ein Fluidstopp in einer der Förderleitungen 21 oder 31 auftritt, so steigt der Druck in dem jeweiligen Druckinfusionsapparat 20 bzw. 30, der von dem dort installierten Drucküberwachungssystem erkannt wird, woraufhin sich dieser Druckinfusionsapparat abschaltet und ein entsprechendes Signal an die Überwachungseinrichtung 1 abgibt, der daraufhin ebenfalls die Schwerkraftinfusionseinrichtung 10 durch Schließen des Ventils 14 und den noch nicht gestoppten Druckinfusionsappart 20 bzw. 30 abschaltet.

Sollte die Infusion bei einem Patienten erfolgen, der zusätzlich noch durch weitere Geräte überwacht wird, wie z.B. durch ein Atmungsüberwachungsgerät, so kann dieses über den Alarmeingang 4 angeschlossen werden. Sollte dieses externe Gerät eine Unregelmäßigkeit feststellen, die eine Einstellung der Infusion erforderlich macht, so wird auf den Alarmeingang 4 ein externes Alarmsignal aufgeschaltet, das die Überwachungseinrichtung veranlaßt, ebenfalls das Ventil 14 und die Druckinfusionsapparate 20 und 30 abzuschalten.

### Bezugszeichenliste:

- 1: Überwachungseinrichtung
- 2: Alarmeingang
- 3: Alarmeingang
- 4: Alarmeingang
- 5: Eingabefeld
- 6: Anzeigefeld
- 7: Steuerausgang
- 8: Steuerausgang
- 9: gemeinsame Signalleitung
- 10: Schwerkraftinfusionseinrichtung
- 11: Vorratsbehälter
- 12: Tropfdetektor
- 13: Förderleitung
- 14: steuerbares Ventil
- 15: Signalleitung
- 16: Steuerleitung
- 20: erster Druckinfusionsapparat
- 21: Förderleitung
- 22: Personalrufanschluß
- 23: Signalleitung
- 24: Steuereingang
- 25: Steuerleitung
- 30: zweiter Druckinfusionsapparat
- 31: Förderleitung
- 32: Personalrufanschluß
- 33: Signalleitung
- 34: Steuereingang
- 35: Steuerleitung
- 40: Dreiwegehahnbank
- 41: Patientenleitung

## Patentansprüche

1. Vorrichtung zur dosierten, kontinuierlichen, gleichzeitigen Infusion von mehreren Infusions- oder Medikamentenlösungen mit mindestens einer Schwerkraftinfusionseinrichtung, in deren zugeordneter Förderleitung ein über einen Steuereingang steuerbares Ventil vorgesehen ist, und/oder mit mindestens einem Druckinfusionsapparat mit einem Alarmausgang sowie mit einer mit dem Steuereingang des Ventils und/oder mit dem Alarmausgang des Druckinfusionsapparates elektrisch verbundenen Überwachungseinrichtung mit mindestens einem Alarmeingang,
dadurch gekennzeichnet,
daß ein erster Alarmeingang (3) der Überwachungseinrichtung (1) mit einem der Schwerkraftinfusionseinrichtung (10) zugeordneten Tropfendetektor (12) und/oder ein zweiter Alarmeingang (2) der Überwachungseinrichtung (1) mit dem Alarmausgang (22, 32) des mindestens einen Druckinfusionsapparates (20, 30) verbunden ist und
daß die Überwachungseinrichtung (1) bei Detektion einer Abweichung von einer vorgegebenen Tropfrate oder beim Anliegen eines Alarmsignals eines Druckinfusionsapparates (20, 30) alle noch nicht desaktivierten Infusionseinrichtungen (10, 20 oder 30) durch Schließen des Ventils (14) und/oder durch Abschalten des jeweiligen Druckinfusionsapparates (20, 30) desaktiviert.

2. Vorrichtung nach Anspruch 1, dadurch gekennzeichnet, daß die Überwachungseinrichtung (1) die Infusionseinrichtungen (10, 20, 30) bei Detektion einer innerhalb einer vorgegebenen Zeitspanne anhaltenden Abweichung von einem vorgegebenen Tropfratenbereich der Schwerkraftinfusionseinrichtung desaktiviert.

3. Vorrichtung nach Anspruch 2, dadurch gekennzeichnet, daß an der Überwachungseinrichtung (1) die vorgegebene Zeitspanne von 10 bis 99 sec und der Tropfratenbereich einstellbar sind.

4. Vorrichtung nach einem der Ansprüche 1 bis 3, dadurch gekennzeichnet, daß der zweite Alarmeingang (2) der Überwachungseinrichtung (1) an den Personalrufanschluß des Druckinfusionsapparates (20, 30) angeschlossen ist.

5. Vorrichtung nach einem der Ansprüche 1 bis 4, dadurch gekennzeichnet, daß ein weiterer Alarmeingang (4) der Überwachungseinrichtung (1) an eine externe Einrichtung zum Aufschalten einer zusätzlichen fremden Alarmbedingung angeschlossen ist, die die Überwachungseinrichtung (1) ebenfalls veranlaßt, alle Infusionseinrichtungen (10, 20, 30) zu desaktivieren.

6. Vorrichtung nach Anspruch 5, dadurch gekennzeichnet, daß die externe Einrichtung ein in einer Patientenleitung (41) angeordneter Druckdetektor ist, dessen Ansprechdruck vom Anwender einstellbar ist.
